# EUROPEAN PATENT APPLICATION

(11) **EP 1 576 983 A1**
(43) Date of publication of application: **21.09.2005**
(21) Application number: 04075847.6
(22) Date of filing: 17.03.2004
(51) Int. Cl.: A61N 1/16

(54) **Method, arrangement and unit for sanitising electromagnetic radiation**

(71) Applicant: Bergs, Godefridus Hubertus Henricus, 6093 NR Heythuysen (NL)
(72) Inventor: Bergs, Godefridus Hubertus Henricus, 6093 NR Heythuysen (NL); Bergs, Engelbertus Andreas, 6093 JB Heythuysen (NL)
(74) Representative: Dohmen, Johannes Maria Gerardus

(57) **Abstract**

The present invention relates to a method of sanitising electromagnetic radiation released from a terrestrial surface. The method comprises installation of a sanitation unit having a operating direction for sanitising said electromagnetic radiation. The method further comprising the steps of positioning said sanitation unit with its operating direction aligned with a propagation direction of said electromagnetic radiation and fixing said sanitation unit relative to said terrestrial surface with its operating direction aligned with said propagation direction.

## Description

### Field of the invention

The present invention relates to a method of sanitising electromagnetic radiation comprising the installation of a sanitation unit relative to a terrestrial surface having an operation direction for sanitizing electromagnetic radiation.

In a further aspect thereof, the invention relates to an arrangement for installing a sanitation unit relative to a terrestrial surface having an operation direction for sanitizing electromagnetic radiation.

In yet another aspect of the present invention, the invention relates to a sanitation unit for use with the method and arrangement of the invention.

### Background of the invention

Numerous studies and investigations have been conducted in order to determine the damaging effects of electromagnetic radiation on the health of living organisms. It has been observed that in some cases the health of living organisms is badly influenced by the presence of electromagnetic radiation, such as terrestrial radiation and/or iatrogenic or man-made radiation. According to studies conducted regarding this subject, it seems to be that it is not the radiation itself which is damaging to the health, but in particular, the damage is caused by certain electromagnetic components carried along with and present within the radiation. The "main" electromagnetic component of the radiation may function as a carrier wave for certain electromagnetic components having a bad influence on certain parts of the body of, for instance, a human being.

It has, for instance, been observed that electromagnetic radiation in a frequency range of a few gigahertz may function as a carrier wave for other electromagnetic radiation components which may have an influence on living organisms. If these other electromagnetic radiation components are out of balance, in the sense that the more damaging components are much stronger than other radiation components carried along with the carrier wave, the radiation as a whole may be experienced as bad. Living organism may therefore experience health problems when present in the neighbourhood of a strong source of unbalanced electromagnetic radiation frequently.

With respect to terrestrial radiation, it is believed that submerged water arteries and other sources function as conductors for this electromagnetic radiation such that the intensity of the electromagnetic radiation increases in the neighbourhood of said water arteries and said sources. The water arteries and other sources therefore effectively provide a distribution system for electromagnetic radiation. Other sources of radiation that are believed to be harmful are GSM radiation and microwave radiation, typically having frequencies in the same frequency range.

Local increases in radiation intensity of electromagnetic radiation may, as explained above, cause harmful and undesired effects. It has been observed that electromagnetic radiation may be the cause of insomnia and other sleeping disorders. Also, the presence of electromagnetic radiation may badly influence the one's ability to concentrate and may even disturb the proper functioning of organs of living organisms. In this context it should be understood that every organ or body part of an organism, such as a plant, animal or human being, is characterized by a certain resonance frequency (and associated resonance wavelength). The resonance wavelengths of organs are often in the range of a few tenths of centimetres, e.g. 15.65 cm for a human heart. Each part of a body is therefore influenced by radiation of a certain frequency.

In any case, the presence of unbalanced electromagnetic radiation is not desired, especially in rooms and buildings where many people spend most of their time, such as in peoples homes and in office buildings. The effects are even worse if one's body is exposed to electromagnetic radiation in such a way that the body absorbs the radiation relatively easy. With terrestrial radiation which is released from the terrestrial surface, this is for instance the case when a person is lying in bed, such that the human body remains in a laid down position and is exposed over a relatively large area. Attempts have been made to neutralise electromagnetic radiation in bedrooms and workrooms, in order to undo their effect.

Therefore, several solutions have been provided in the form of devices that either absorb, deflect or neutralise the electromagnetic radiation in one or another way. These devices are often constituted by configurations of materials that are known to be sensitive to electromagnetic radiation, have the ability to influence the radiation path, or which are known absorbers of electromagnetic radiation. The devices are preferably installed at location where the intensity of the electromagnetic radiation is relatively high, or at location where the electromagnetic radiation is expected to cause harmful effects, such as in bedrooms, workrooms, etc.

A problem with the installation of the devices, is that electromagnetic radiation is difficult to detect. Radiation sources may be detected using a dowsing rod or dowser, however this method of detecting radiation has turned out to be very inaccurate.

Use is sometimes made of a lecher antenna for detecting the electromagnetic radiation. This is a more accurate way of measuring electromagnetic radiation in the microwave range, such as terrestrial radiation and GSM radiation, and has the additional benefit that it enables the operator to sense electromagnetic radiation of a specific wavelength.

Although locating the radiation source or the radiation maximum can be performed more accurate, it has been observed that prior art methods of neutralising the radiation are often insufficiently effective. The sphere of influence of these devices is often limited and/or the devices are somehow incorrectly installed and are therefore ineffective.

### Summary of the invention

It is an object of the present invention to provide an effective method of sanitizing electromagnetic radiation, such as to reduce the effects thereof.

This and other objects are achieved by the present invention in that there is provided a method of sanitising electromagnetic radiation, comprising installation of a sanitation unit relative to a terrestrial surface having an operating direction for sanitising said electromagnetic radiation, said method comprising the steps of positioning said sanitation unit with its operating direction aligned with a propagation direction of said electromagnetic radiation and fixing said sanitation unit relative to said terrestrial surface with its operating direction aligned with said propagation direction.

The invention is based on the insight that sanitising units often have an operating direction, and that since electromagnetic radiation has an origin and a propagation direction, not only the exact placement of the sanitation unit is important but also the alignment to the radiation. It has been observed that most sanitation units comprise a operation direction, for which sanitation of the electromagnetic radiation is most effective. By aligning said operation direction with a propagation direction of the electromagnetic radiation, the effects of the sanitation unit are maximised, increasing the sphere of influence of the sanitation unit. Therefore, by using the method of the present invention and fixing the sanitation unit relative to the terrestrial surface with its operation direction aligned with the propagation direction, electromagnetic radiation can effectively be sanitised.

In another embodiment wherein said electromagnetic radiation comprises a left circularly polarized component and a right circularly polarized component, said sanitation unit is arranged for sanitising said electromagnetic radiation such that the intensity of said left circularly polarized component relative to said right circularly polarized component is substantially 5:8.

It has been observed that along with the electromagnetic carrier wave, additional electromagnetic energy is transported in the form of electromagnetic sub-components. These sub-components may comprise left circularly polarized components and right circularly polarized components. The left circularly polarized components are observed to be harmful. It has further been observed that the least harmful, ideal proportion between left and right circularly polarized components is approximately 5:8. Sanitising of electromagnetic radiation is therefore best performed using a sanitation unit which is arranged for sanitising said electromagnetic radiation such that the intensity of said left circularly polarized component relative to said right circularly polarized component is substantially 5:8.

In another embodiment, wherein said surface comprises a plurality of energy lines, and wherein said electromagnetic radiation is released from said terrestrial surface substantially through said energy lines, said sanitation unit is fixed relative to said terrestrial surface substantially on said energy lines.

It is believed that the terrestrial radiation finds its origin in the earth magnetic field. Studies have lead to the conclusion that a dense network of energy lines across the terrestrial surface defines lines where the intensity of the terrestrial radiation reaches a maximum. It may therefore be understood that the energy lines define lines through which electromagnetic radiation is released from the terrestrial surface. By placing the sanitation unit close to the energy lines, terrestrial radiation can be sanitised more effectively.

In a preferred embodiment of the present invention, wherein said plurality of energy lines form at least one grid on the terrestrial surface, and wherein said grid forms a plurality of junctions, said sanitation unit is fixed on a junction of said grid.

In principle, the energy lines form two different dense grids across the terrestrial surface. Energy lines of the orthogonal grid are running from north to south and from west to east, forming junctions where north-south energy lines intersect with west-east energy lines. The other dense grid of energy lines is formed by the diagonal grid, of which the energy lines either run from northwest to southeast or from southwest to northeast, forming again junctions at their intersections. In each junction, the release of electromagnetic radiation from the energy lines intersecting at the junction adds up to a local maximum. By fixing the sanitation unit substantially on a junction of said grid, a major source of electromagnetic radiation in the area surrounding the junction is effectively sanitised.

In a preferred embodiment of the present invention, wherein electromagnetic radiation is released from said energy lines on said terrestrial surface in a propagation direction having a propagation angle relative a normal vector perpendicular to said terrestrial surface, said operation direction is aligned by establishing an alignment angle substantially equal to said propagation angle. Yet in another preferred embodiment, wherein said sanitation unit is fixed near or on a junction of any of the abovementioned grids, said alignment angle is established by combining the propagation angles of said energy lines intersecting at said junction.

A characteristic of the electromagnetic radiation is that it propagates from the surface of the earth under certain angles relative to the energy lines and the earth surface. The angle under which the electromagnetic radiation is released from the surface is dependent on whether the energy line belongs to the orthogonal grid or to the diagonal grid.

On the energy lines of the orthogonal grid, electromagnetic radiation is released from each energy line, propagating under an angle of approximately 25 degrees relative to the normal vector of the earth surface. Here, the angle defines the deviation of the propagation direction relative to the normal vector, wherein said deviation is directed perpendicular to the direction of the energy line relative to said normal vector. This angle of approximately 25 degrees is valid for both the north-south energy lines and the west-east energy lines. In each junction, the combined propagation angle of electromagnetic radiation, therefore constitutes an angle of 36.7 degrees, as can be calculated using Pythagoras' theorem.

The deviation of the propagation direction relative to the normal vector of the earth surface for the energy lines in the diagonal grid equals approximately 12.5 degrees. Similar to the above, the deviation angle at each junction of energy lines within the diagonal grid, equals approximately 17.8 degrees.

The propagation angles of the electromagnetic radiation released from each energy line therefore combine at a junction of energy lines, such that the resulting electromagnetic radiation from both energy lines radiated at the junction has a propagation angle equal to the combination angle of the propagation angles of both components of the electromagnetic radiation (relating to the respective energy lines). The sanitation unit should therefore be installed in such a way that it is aligned to the propagation direction at the junction (i.e. the combined angle of the two components of the electromagnetic radiation adding up at the junction), in order to be most effective. It will be understood that the sanitation unit may also be installed near an energy line, but not especially near a junction.

In another embodiment of the invention said alignment angle is substantially equal to any of a group comprising 12.5 degrees, 17.8 degrees, 25 degrees, 36.7 degrees or 0 degrees. It should be understood that the propagation direction at a junction in the orthogonal grid is approximately 36.7 degrees, the propagation direction at a junction in the diagonal grid is approximately 17.8 degrees, and near water arteries, a propagation direction having an alignment angle of 0 degrees is preferably used since the propagation direction near water arteries is undecided and will on average be parallel to the normal vector, perpendicular to the earth surface. If a sanitation unit is to be installed near energy lines of the orthogonal grid, but not at a junction of energy lines, so somewhere along an energy line, the alignment angle should be 25 degrees relative to the normal vector perpendicular to the earth surface. Further to this, if a sanitation unit is to be installed near energy lines of the diagonal grid, not at a junction of energy lines but somewhere along an energy line, the alignment angle should be 12.5 degrees relative to the normal vector perpendicular to the earth surface.

Note further that the deviation direction, the direction wherein the operation direction of the sanitation unit should be angled using the alignment angle relative to the normal vector (i.e. the direction of the mathematical projection of the operation direction onto the terrestrial surface), is perpendicular to the direction of the energy lines. If installed at a junction, where the energy lines perpendicularly cross each other, this deviation direction makes an angle of 45 degrees with each of the energy lines.

Both from an aesthetic as well as from a practical point of view, the sanitation unit is preferably submerged beneath the surface of the earth. Submerging the sanitation unit keeps it fixed to the predetermined alignment angle without using any additional fixing means.

In a preferred embodiment of the present invention, the sanitation unit comprises an elongated rod. It has been observed that sanitation units comprising the shape of an elongated rod, or a similar shape, effectively sanitises the electromagnetic radiation in their operation direction. The operation direction of the elongated rod of the present invention is observed to be the axial direction of the rod.

According to another preferred embodiment, the sanitation unit comprises at least one electrically conductive material. The electromagnetic energy in electromagnetic radiation is best sanitised using electrical conductive materials, such as aluminum, carbon, gold, silver, brass or other conductive materials such as conductive polymers.

Said sanitation unit may be covered using a covering layer, which protects the sanitation unit against corrosion and influences of moisture and of the weather, when said sanitation unit is installed, e.g. submerged in the ground or above the ground fixed to a wall. Apart from protection against corrosion, another, maybe even more important, benefit of using a covering layer is that it may increase the effects of the sanitation unit. This is due to the fact that a covering layer may concentrate or bundle the electromagnetic radiation, as has been observed for some materials and some configurations.

A particularly beneficial group of materials that may be used for forming the covering layer are polymeric materials such as polyvinylchloride (PVC), as is used in an embodiment of the invention. In particular for PVC, it has been observed that the effects of a sanitation unit may be increased roughly by a factor 200. The most beneficial and effective configuration for increasing the effects of the sanitation unit is a covering layer which annularly covers the conductive core of the sanitation unit (e.g. elongated rod), whilst both ends of the unit are left open. Although in this configuration the sanitation units are observed to be highly efficient, a disadvantage of this configuration is that the conductive core is still prone to corrosion at both of its open ends.

Therefor, in another embodiment, the covering layer enclosing an elongated rod is closed on either end of the rod, sealing the sanitation unit for protection against moisture. The covering layer, as well as the closure on either end of the rod, may comprise said polymeric material, such as polyvinylchloride. It will be appreciated by the person skilled in the art, that such a covering layer may effectively shield the sanitation unit for protection against moisture and influences of the weather, whilst it still carries most of the benefits of using PVC as a covering layer.

In accordance with a second aspect of the present invention, there is provided an arrangement for installing a sanitation unit relative to a terrestrial surface, said sanitation unit having a operating direction for sanitising electromagnetic radiation, comprising means for receiving said sanitation unit, means for aligning said operating direction of said sanitation unit with a propagation direction of said electromagnetic radiation, and means for fixing said sanitation unit relative to said terrestrial surface with its operating direction aligned with said propagation direction.

Such an arrangement may advantageously be used with the method of the present invention, for performing the installation of the sanitation units and for achieving the benefits of the present invention.

In an embodiment of said second aspect, said means for receiving said sanitation unit comprises at least one container, and said container further comprises said means for aligning said operation direction with said propagation direction. In particular, said means for aligning said operation direction, in an embodiment of the invention, are arranged for positioning said orientation unit with its operation direction under an alignment angle relative to said terrestrial surface. It may be appreciated that such an arrangement can beneficially be used for carrying out the method of the present invention.

According to an embodiment of the present invention said arrangement further comprises at least one adjustable support means for levelling said arrangement relative to said terrestrial surface. Since the sanitation unit must be aligned accurately to the propagation direction of the electromagnetic radiation, it is important to provide the arrangement with means for levelling the arrangement to the terrestrial surface, such that irregularities in the underground on which the arrangement may be placed, may be anticipated or compensated in order to avoid deviations in the alignment angle caused by irregularities in the underground. Therefore, the arrangement may further comprise levelling means for verifying levelling of said arrangement relative to said terrestrial surface.

In another embodiment of the present invention said arrangement further comprises positioning means for verifying placement of said arrangement relative to one or more energy lines on said terrestrial surface, said energy lines substantially releasing said electromagnetic radiation from said surface.

By including positioning means with the arrangement of the present invention, the exact placement of the arrangement can effectively be verified, such that it will be much easier to place the arrangement on or in the vicinity of an energy line. Said positioning means may for instance include a compass, a global positioning system (GPS) or any other positioning means. It will be appreciated that by using a compass, the alignment of the arrangement in a north-south-direction or a west-east-direction or northeast-southwest-direction or southwest-northeast-direction can effectively be verified. Using a global positioning system, the same benefits can be achieved as with using a compass, although the additional benefit is that the exact location on the earth surface and can be easily verified.

According to another embodiment of the present invention, said means for receiving said sanitation unit are arranged for maintaining said sanitation unit at a fixed angle relative to said terrestrial surface, after said arrangement is levelled with said terrestrial surface. It will be appreciated, that the means for receiving the sanitation unit, e.g. the container mentioned above, may be mounted on the arrangement at a fixed angle relative to the levelling of said arrangement. It will be much easier to carry out the method, since for installation of the sanitation unit, the operator merely needs to lign up the arrangement relative to the energy lines and/or verify the position, and level the arrangement relative to the terrestrial surface using the adjustable support means. Since the angle relative to the levelling of the arrangement is fixed, after installing the arrangement correctly, the alignment angle will automatically be achieved.

Note that this is in particular possible since the propagation directions from the energy lines are known, and therefore the possible alignment angles, or at least the correct alignment angles in most of the cases, can be calculated from these known propagation directions. In particular these known alignment angles will either be 12.5 degrees, 17.8 degrees, 25 degrees, 36.7 degrees or 0 degrees (the latter if the propagation direction of the electromagnetic radiation is perpendicular to the terrestrial surface, thereby forming an alignment angle of 0 degrees with a normal vector perpendicular to the terrestrial surface).

Note that according to another embodiment of the present invention, said means for receiving said sanitation unit comprises a hollow tube. This is in particular beneficial in cases wherein the sanitation unit is an elongated rod or comprises a similar form, which easily fits within said hollow tube. After placement of the arrangement, verifying its location and alignment relative to at least one energy line, and levelling the arrangement, the sanitation unit (e.g. the elongated rod), may for instance be pushed down through the hollow tube into the earth surface such that it immediately becomes fixed at its alignment angle as soon as it is submerged in the ground.

According to a third aspect of the present invention said invention is directed to a sanitation unit for use with a method according to the present invention, said sanitation unit comprising an elongated rod of electrically conductive material.

Said electrically conductive material may, according to an embodiment of this third aspect, comprise at least one of a group comprising copper, aluminium, carbon, gold, silver, brass or a conductive polymer.

Said sanitation unit may further comprise a covering layer in order to increase the effects of the sanitation unit. As explained hereinbefore, the effects of the sanitation unit can be increased efficiently if said covering layer comprises, according to another embodiment of the invention, a polymer such as polyvinylchloride (PVC). Further to this, the covering layer may be an annular layer enclosing the conductive material of the sanitation unit, whilst both ends of the sanitation unit are left uncovered by the covering layer. This will efficiently increase the effects of the sanitation unit. A disadvantage of this configuration however, is that the conductive material may be prone to corrosion at its open ends.

Therefore, in addition said covering layer may shield the sanitation unit, or at least the parts of the sanitation unit that are prone to corrosion, from the effects of moisture from the soil of the terrestrial surface wherein it is submerged, or other weather influences in case the sanitation unit is installed above the ground in a humid area or outdoor. According to another embodiment of this invention, said elongated rod is therefore fully enclosed by said covering layer, including the ends of the rod.

It will further be appreciated that, according to an embodiment of the invention, the sanitation unit is arranged for sanitising electromagnetic radiation such that the intensity of a left circularly polarized component comprised by said electromagnetic radiation relative to a right circularly polarized component comprised by said electromagnetic radiation is substantially 5:8. As explained above, possible harmful effects of the electromagnetic radiation can effectively be undone by balancing the left and right circularly polarised electromagnetic radiation components thereof in the abovementioned proportions.

The present invention will be further elucidated by a description and drawings, referring to a preferred embodiment thereof. The invention is not limited to the embodiments disclosed, which are provided for explanatory purposes only.

### Brief description of the drawings

Figure 1 is a schematic drawing of a planet comprising an orthogonal grid of energy lines.
Figure 2 is a schematic drawing of a junction of energy lines showing a propagation direction of electromagnetic radiation.
Figure 3 illustrates an arrangement according to the present invention.
Figure 4 shows a sanitation unit according to the present invention.
Figure 5 shows a cross section of the sanitation unit of figure 4.
Figure 6 is a schematic drawing of a method according to the present invention.

### Detailed description of the drawings

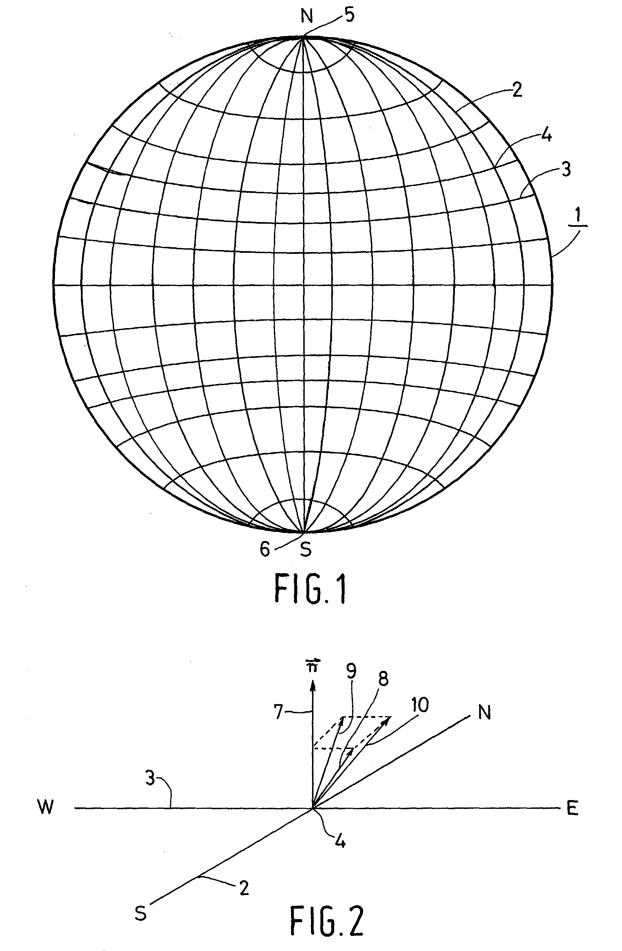

Figure 1 shows a planet 1 having a magnetic north pole 5 and a magnetic south pole 6. On the surface of the planet 1, a plurality of energy lines 2, 3 are running north-south (such as energy line 2) and east-west (such as energy line 3).

At each energy line, the outgoing flux of electromagnetic radiation reaches a local maximum. Therefore, at each junction (such as junction 4) the outgoing flux of terrestrial energy from the energy lines 2, 3 intersecting at said junction 4, reaches a maximum due to the fact that the electromagnetic radiation energy from both energy lines adds up at the junction 4.

As is schematically indicated in figure 2, the overall propagation direction of the energy flux from each energy line forms an angle with the normal vector 7, which normal vector 7 is perpendicular to the earth surface. For instance, propagation direction vector 8 in figure 2, forms an angle with the normal vector 7 and deviates from the normal vector 7 in a direction perpendicular to the direction north-south. The deviation angle or propagation angle of propagation vector 8 for a north-south energy line from the orthogonal grid, equals approximately 25 degrees. The propagation direction indicated by the propagation vector 9, which is the propagation direction for electromagnetic radiation released at west-east energy line 3, deviates from the normal vector 7 perpendicular to the earth surface in a direction perpendicular to the west-east direction as indicated by vector 9 in figure 2. The propagation angle of vector 9 approximately equals 25 degrees, similar to the propagation angle of the north-south energy lines 2.

In view of the above, the propagation angle of the resulting electromagnetic radiation from the junction 4, as indicated by vector 10, deviates from the normal vector 7 under an angle of 36.7 degrees in the direction as indicated in figure 2. The above can be calculated using the well known Pythagoras theorem.

Similar to the energy lines of the orthogonal grid, a second set of energy lines running from northwest to southeast and running from southwest to northeast forms the diagonal grid. Similar to the above, the propagation angle of the electromagnetic radiation released by each of the energy lines in the diagonal grid deviates from the normal vector perpendicular to the earth surface with a propagation angle of 12.5 degrees, thereby combining in the junction of two intersecting energy lines with a propagation angle of 17.8 degrees.

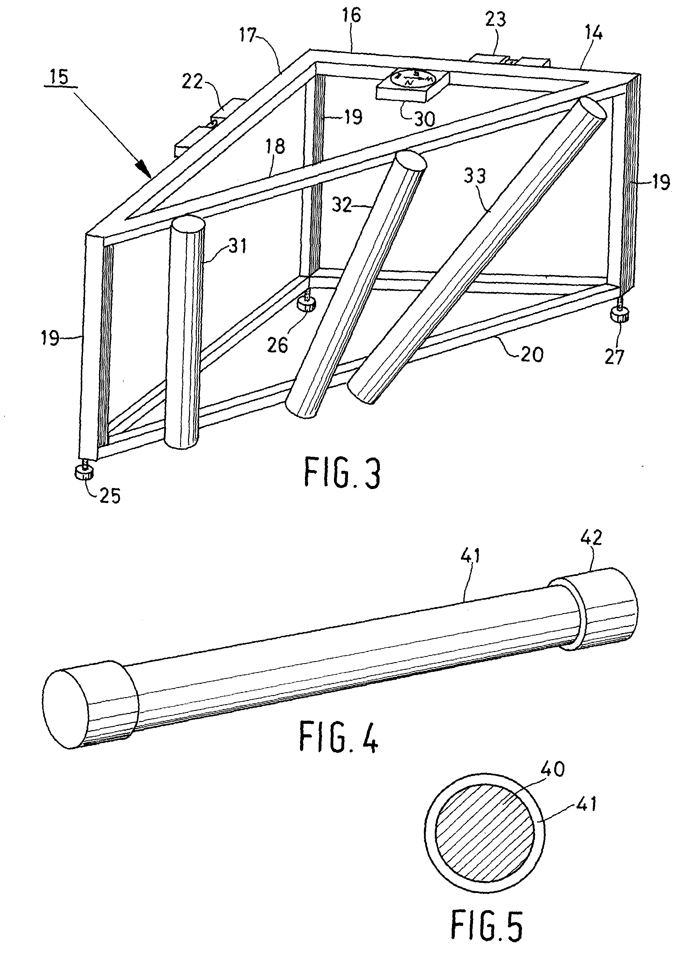

Figure 3 shows an arrangement 15 according to the present invention, comprising an upper support construction 14 and a lower support construction 20 connected by a set of vertical legs 19. The upper and lower support constructions (14, 20) are comprised of two perpendicular support bars 16 and 17, and a diagonal support bar 18 connecting the ends of support bars 17 and 16. The support structures thereby form two triangles (14, 20) connected by vertical legs 19.

The support structure further comprises a plurality of adjustable support means 25, 26 and 27, each comprised of tapped cylindric means which are screwably connected to the lower support structure 20 preferably near or underneath each leg 19. Underneath the tapped cylindrical means there is provided a support foot as indicated in figure 3.

By screwing and unscrewing each of the adjustable support means 25, 26 and 27, each of the legs 19 can be adjusted in the vertical direction, such that the arrangement as a whole can be levelled parallel to the terrestrial surface. Note that the arrangement comprises on each of the bars 16 and 17 a level 22 and 23 for verifying the levelling in two directions. The adjustable support means 25, 26, and 27 enable the operator of the arrangement to level the arrangement, and the levels 22 and 23 connected to the arrangement enable the operator to verify the levelling of the arrangement. Note that each level 22 and 23 is a regular level known to the persons skilled in the art, for instance comprised of a transparent, slightly curved tube filled with a coloured liquid, such as coloured water, further comprising a small bubble of air and indicator lines to indicate levelling of the level.

The arrangement further comprises positioning means in the form of a compass 30. The compass 30 enables an operator of the arrangement to align either of the bars 16 or 17 to an energy line of a grid, such as the north-south energy lines and the west-east energy lines of the orthogonal grid. After the operator of the arrangement has determined the junction of energy lines, for instance by determining the location where the energy flux of electromagnetic radiation reaches a local absolute maximum, the operator may place the arrangement at said maximum and align the perpendicular bars 17 and 16 of the arrangement to the energy lines by rotating the arrangement such that the magnetic arrow of the compass indicates west, north, east or south. If the arrangement is to be aligned with the energy lines of the diagonal grid, the arrangement should be rotated such that the needle of the compass indicates either northwest, northeast, southeast or southwest.

In an improvement of the arrangement of figure 3, there is provided a global positioning system (GPS) (not shown), having the additional benefit that the arrangement may accurately be placed on a certain location under terrestrial surface, whilst the GPS can also be used as compass.

After having the arrangement placed at a correct location, and after aligning the bars 15 and 17 to the energy lines using the compass 30 or GPS (not shown), the adjustable support means 25, 26 and 27 may be adjusted in order to improve the levelling of the arrangement, which can be verified by the operator using the levels 22 and 23 as indicated above.

As soon as the arrangement is placed correctly and levelled with the terrestrial surface, the sanitation unit may be easily installed, e.g. submerged in the ground, by placing a sanitation unit in any of the containers 31, 32 or 33 as desired, and pushing the sanitation unit through any of the containers 31, 32 and 33 into the terrestrial surface. In case the terrestrial surface is too rigid for the operator to be able to push the sanitation unit into the ground, the operator may use a tool, such as a drill for making a hole first. It will be appreciated that the arrangement may be equipped with or provided with such a tool, such as a soil drill or a tool for forcing the sanitation unit into the ground.

Note that the containers 31, 32 and 33 have a fixed alignment angle with the terrestrial surface after the arrangement is levelled. For instance container 31 is placed perpendicular to the terrestrial surface, thereby forming an alignment angle of 0 degrees with the normal vector to the terrestrial surface. Container 32 has an alignment angle of 17.8 degrees with the normal vector perpendicular to the earth surface. Further to this, container 33 has an alignment angle of 36.7 degrees relative to the normal vector perpendicular to the terrestrial surface. Note that container 32 may specifically be used at junctions of energy lines of the diagonal grid, where the propagation angle of the electromagnetic radiation equals 17.8 degrees. Similarly, container 33 may be used for installing sanitation units at junctions between energy lines of the orthogonal grid, where the propagation direction of the electromagnetic radiation has an angle relative the normal vector perpendicular to the earth surface of 36.7 degrees. Container no. 31 may be used in situations where the propagation direction is either undetermined, or on average perpendicular to the earth surface, such as near or above water arteries.

For the purpose of installing sanitation units on the energy lines of the orthogonal and diagonal grids, in between junctions thereof, so at any point along an energy line, containers may be fixed to the arrangement having an angle of 12.5 degrees for the diagonal grid, or 25 degrees for the orthogonal grid. For the purpose of comprehensiveness of the drawings, the latter two containers are not shown in figure 3. However, these containers may be installed on any suitable point on the arrangement, e.g. fixed to bars 16 and/or 17 of the arrangement.

It will be appreciated that any one of the containers may be rotatably fixed to the arrangement, such as to be able to adjust the alignment angle. In order to verify the alignment angle in an arrangement where one of the containers is rotatably fixed, the arrangement may comprise a scale indicator (not shown) for indicating the angle of the container relative to the arrangement.

Figure 4 shows a sanitation unit that may be used in combination with the arrangement of figure 3. The sanitation unit, of which a cross section is illustrated in figure 5, is comprised of a conductive core 40, covered by a covering layer 41. The sanitation unit takes the form of an elongated rod, and the covering layer 41 is closed on each end of the rod with the closure 42. As explained hereinbefore, the use of a covering layer may increase the effects of the sanitation unit. This will especially be the case if a covering layer comprising polyvinylchloride (PVC) is used. Note that the closure 42 is optional, as the effects of the sanitation units are increased more if a sanitation unit with open, uncovered ends is used.

The closure 42 (combined with the covering layer 41) mainly makes sure that the conductive material 40 within the sanitation unit is completely sealed from the outside, such that moisture in the soil of the terrestrial surface will not be able to come into contact with the conductive material 40 in the sanitation unit. The conductive material may for instance be of copper, which is prone to corrosion.

Note that the conductive material 40 of the sanitation unit may comprise any electrically conductive material such as copper, aluminum, carbon, gold, silver, brass and/or a conductive polymer. It has been observed that the sanitation unit works well if it comprises any one or more of these materials.

In the above configuration, it has been observed that the sanitation unit is arranged for sanitising electromagnetic radiation such that the intensity of a left circularly polarized component comprised by said electromagnetic radiation relative to a right circularly polarized component comprised by said electromagnetic radiation is substantially 5:8. Possible harmful effects of the electromagnetic radiation can effectively be undone by balancing the left and right circularly polarised electromagnetic radiation components thereof in the abovementioned proportion.

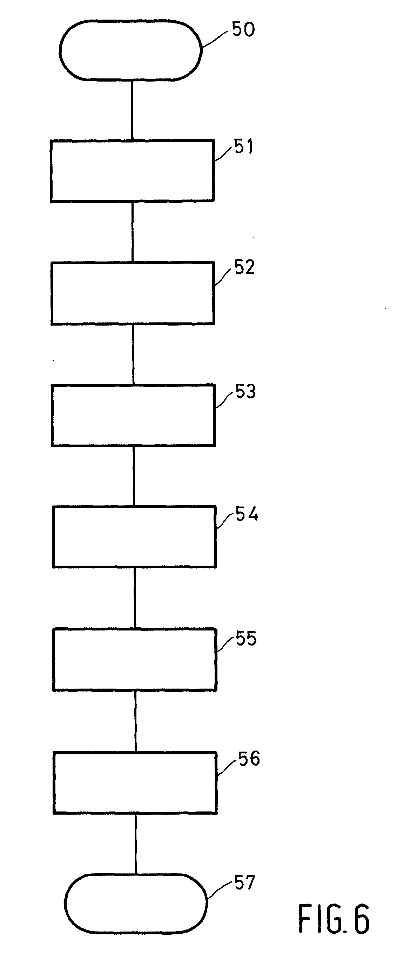

In figure 6 a method according to the present invention is schematically illustrated, wherein reference signs 50 and 57 indicate the start (50) and end (57) of the method respectively. The method may comprises a step of measuring or locating the energy maximum which indicates either an energy line or a junction of energy lines. Said measuring of the energy maximum is indicated by step 51. In step 52, if the energy maximum has been found, the arrangement is placed at a location of the energy maximum. After the arrangement for installing the sanitation unit has been placed at a correct location, the arrangement is to be aligned in step 53 relative to the local energy lines, which either run north-south, west-east, northwest-southeast or southwest-northeast. This may be achieved by using a compass, such as compass 30 in figure 30 or a global positioning system (GPS) (not shown).

In step 54, the arrangement is levelled with the terrestrial surface using the adjustable support means and the optional levels comprised by the arrangement.

After having placed the arrangement and after having levelled it to the terrestrial surface, installation of the sanitation unit may be achieved in step 55, by aligning the operation direction of sanitation unit with the propagation direction of the electromagnetic radiation, and subsequently in step 56 by fixing the sanitation angle relative to the terrestrial surface. This last step 56 may for instance be performed by submerging the sanitation unit in the terrestrial surface, optionally by using a soil drill, a pointed pole, or a tool for forcing the sanitation unit into the ground.

For the purpose of comprehensiveness, it is noted here that numerous modifications and the variations of the present invention are possible in the light of the above teachings. It is therefore understood that, within the scope of the appended claims, the invention may be practised otherwise than as specifically described herein.

## Claims

1. Method of sanitising electromagnetic radiation, comprising installation of a sanitation unit relative to a terrestrial surface having an operating direction for sanitising said electromagnetic radiation, said method comprising the steps of positioning said sanitation unit with its operating direction aligned with a propagation direction of said electromagnetic radiation and fixing said sanitation unit relative to said terrestrial surface with its operating direction aligned with said propagation direction.

2. Method according to claim 1, wherein said electromagnetic radiation comprises a left circularly polarized component and a right circularly polarized component, and wherein said sanitation unit is arranged for sanitising said electromagnetic radiation such that the intensity of said left circularly polarized component relative to said right circularly polarized component is substantially 5:8.

3. Method according to any of the previous claims, wherein said terrestrial surface comprises a plurality of energy lines, wherein said electromagnetic radiation is released from said terrestrial surface substantially through said energy lines, and wherein said sanitation unit is fixed relative to said terrestrial surface on said energy lines.

4. Method according to claim 3, wherein said plurality of energy lines form at least one grid on said terrestrial surface, said grid forming a plurality of junctions, and wherein said sanitation unit is fixed on a junction of said grid.

5. Method according to any of the previous claims, wherein said electromagnetic radiation is directed in a propagation direction having a propagation angle relative to a normal vector perpendicular to said terrestrial surface, and wherein said operating direction is aligned by establishing an alignment angle substantially equal to said propagation angle.

6. Method according to claim 5 as dependent on claim 4, wherein said alignment angle is established by combining said propagation angles of said energy lines intersecting at said junction.

7. Method according to any of the claims 5 or 6, wherein said alignment angle is substantially equal to any of a group comprising 12.5 degrees, 17.8 degrees, 25 degrees, 36.7 degrees or 0 degrees.

8. Method according to any of the previous claims, wherein fixing said sanitation unit comprises fixing said sanitation unit submerged underneath said terrestrial surface.

9. Method according to any of the previous claims, wherein said sanitation unit comprises an elongated rod.

10. Method according to any of the previous claims, wherein said sanitation unit comprises at least one electrically conductive material.

11. Method according to claim 10, wherein said electrically conductive material comprises at least one of a group comprising copper, aluminum, carbon, gold, silver, brass or a conductive polymer.

12. Method according to any of the previous claims, wherein said sanitation unit further comprises a covering layer.

13. Method according to claim 12, as dependent on claim 9, wherein said elongated rod is enclosed by said covering layer, and wherein said covering layer being closed on either end of the rod.

14. Method according to any of the claims 12 or 13, wherein said covering layer comprises a polymer, such as polyvinylchloride (PVC).

15. Arrangement for installing a sanitation unit relative to a terrestrial surface, said sanitation unit having a operating direction for sanitising electromagnetic radiation, comprising means for receiving said sanitation unit, means for aligning said operating direction of said sanitation unit with a propagation direction of said electromagnetic radiation, and means for fixing said sanitation unit relative to said terrestrial surface with its operating direction aligned with said propagation direction.

16. Arrangement according to claim 15, wherein said means for receiving said sanitation unit comprises at least one container, said container further comprising said means for aligning said operating direction with said propagation direction.

17. Arrangement according to any of the claims 15 or 16, wherein said means for aligning said operating direction are arranged for positioning said orientation unit with its operating direction under an alignment angle relative to said terrestrial surface.

18. Arrangement according to any of the claims 15-17, further comprising at least one adjustable support means for levelling said arrangement relative to said terrestrial surface.

19. Arrangement according to any of the claims 15-18, further comprising levelling means for verifying levelling said arrangement relative to said terrestrial surface.

20. Arrangement according to any of the claims 15-19, further comprising positioning means for verifying placement of said arrangement relative to one or more energy lines on said terrestrial surface, said energy lines substantially releasing said electromagnetic radiation from said terrestrial surface.

21. Arrangement according to claim 20, wherein said positioning means comprises any of a group comprising a compass, a global positioning system (GPS) or any other positioning means.

22. Arrangement according to any of the claims 15-21, wherein said means for receiving said sanitation unit are arranged for maintaining said sanitation unit at a fixed angle relative to a normal vector perpendicular to said terrestrial surface, after said arrangement is levelled with said terrestrial surface.

23. Arrangement according to claim 22, wherein said fixed angle is substantially equal to any of a group comprising 12.5 degrees, 17.8 degrees, 25 degrees, 36.7 degrees or 0 degrees.

24. Arrangement according to any of the previous claims 15-23, wherein said sanitation unit comprises an elongated rod and wherein said means for receiving said sanitation unit comprises a hollow tube.

25. Sanitation unit for use with a method according to any of the claims 1-14, said sanitation unit comprising an elongated rod of electrically conductive material.

26. Sanitation unit according to claim 25, wherein said electrically conductive material comprises at least one of a group comprising copper, aluminum, carbon, gold, silver, or a conductive polymer.

27. Sanitation unit according to any of the claims 25 or 26, wherein said sanitation unit further comprises a covering layer.

28. Sanitation unit according to claim 27, wherein said elongated rod is fully enclosed by said covering layer, including the ends of the rod.

29. Sanitation unit according to any of the claims 27 or 28, wherein said covering layer comprises a polymer, such as polyvinylchloride (PVC).

30. Sanitation unit according to any of the claims 25-29, wherein said sanitation unit is arranged for sanitising electromagnetic radiation such that the intensity of a left circularly polarized component comprised by said electromagnetic radiation relative to a right circularly polarized component comprised by said electromagnetic radiation is substantially 5:8.
